# EUROPÄISCHE PATENTSCHRIFT

(11) **EP 1 136 084 B1**
(45) Veröffentlichungstag und Bekanntmachung des Hinweises auf die Patenterteilung: **04.10.2017**
(21) Anmeldenummer: 01103992.2
(22) Anmeldetag: 20.02.2001
(51) Int. Cl.: A61L 24/10, A61K 38/48, A61K 47/26, A61K 47/18, A61K 47/02, A61K 47/00, A61P 7/04, A61L 24/04

(54) **Thrombin-Zubereitungen und Verfahren zu ihrer Herstellung**
Compositions containing thrombin and process for their manufacture
Compositions comprenant de la thrombine et procédé pour leur manufacture

(30) Priorität: 18.03.2000 DE 10012732
(43) Veröffentlichungstag der Anmeldung: 26.09.2001
(73) Patentinhaber: CSL Behring GmbH, 35041 Marburg (DE)
(72) Erfinder: Metzner, Hubert, Dr., 35041 Marburg (DE); Schneider, Heinrich, 35094 Lahntal (DE)

(56) Entgegenhaltungen:
- EP-A- 0 439 156
- EP-A- 0 444 692
- EP-A- 0 543 178
- EP-A1- 1 136 084
- EP-A2- 0 796 623
- WO-A-99/00356
- WO-A-99/37304
- WO-A1-98/44942
- WO-A2-99/66031
- DE-A- 19 853 033
- DE-A1- 19 531 637
- US-A- 4 696 812
- US-A- 5 219 328
- US-A- 6 013 620
- K O Eriksson: "Hydrophobic Interaction Chromatography" In: "Protein Purification Principles, High Resolution Methods and Applications", 1 January 1989 (1989-01-01), VCH Publishers, XP055195077, pages 207-215,
- "Hydrophobic Interaction Chromatography (HIC)" In: "Principles and Techniques of Practical Biochemistry - 4th Ed.", 1 January 1994 (1994-01-01), Cambridge University Press, New York, XP055195064, pages 491-492,
- K G Mann: "Prothrombin and Thrombin" In: "Hemostasis and Thrombosis, Basic Principles and Clinical Practice, 3rd Ed.", 1 January 1994 (1994-01-01), JB Lippincott Company, Philadelphia, XP055195082, pages 184-199,

## Beschreibung

Der Gegenstand der Erfindung ist eine im flüssigen Zustand stabile Thrombin-Zubereitung, die sich durch eine hohe Reinheit und Virussicherheit auszeichnet, sowie ein Verfahren zu ihrer Herstellung.

Seitdem es gelungen ist, Thrombin kommerziell herzustellen, haben sich hierfür mehrere Anwendungen ergeben. Als Hauptanwendungen sind derzeit neben diagnostischen Zwecken der Einsatz als lokales Hämostyptikum oder als Komponente eines Gewebeklebers zusammen mit einer Fibrinogen-haltigen Komponente zu nennen. Voraussetzung für den Einsatz von Thrombin für medizinische Zwecke ist, dass es dem Arzt als stabiles Präparat zur Verfügung gestellt werden kann, welches eine hohe Virussicherheit aufweist und möglichst keine unwirksamen Neben- oder Abbauprodukte des Thrombins bzw. anderer Faktoren enthält.

Zahlreiche Methoden zur Stabilisierung von Thrombin sind bereits vorgeschlagen worden. So ist aus der japanischen Patentanmeldung Nr. 56-39782 ein Verfahren bekannt, bei dem organische Mono- oder Polycarbonsäuren und/oder Mono- oder Polyhydroxycarbonsäuren zur Herstellung von stabilen wässrigen Lösungen von Thrombin eingesetzt werden. Aus der japanischen Patentanmeldung Nr. 57-18985 ist Albumin als Stabilisator für Thrombin und aus der japanischen Patentanmeldung Nr. 62-106028 eine Pufferlösung als Stabilisator bekannt. In der europäischen Patentanmeldung 0 302 754 werden ein Zucker und eine Aminosäure, bevorzugt in einer Konzentration von 1 bis 10 Gew.%, als Stabilisatoren für Thrombin-Lösungen vorgeschlagen.

Des weiteren ist aus der deutschen Offenlegungsschrift 31 22 926 eine lagerfähige Thrombin-Zubereitung bekannt, die neben Natriumchlorid mehrwertige Alkohole mit 3 bis 6 Kohlenstoffatomen, schwefelfreie Aminosäuren und Polyethylenglykol zur Herstellung von Thrombinlösungen beschreibt. Schließlich ist auch in der europäischen Patentanmeldung 0 221 700 eine bei einem pH-Wert von 5 bis 8 gepufferte Thrombin-Zubereitung beschrieben, die ggfs. Natriumchlorid und eine Polyhydroxyverbindung enthalten kann.

Gepufferte und stabilisierte Thrombinlösungen sind auch aus einer Veröffentlichung von J. Chabbat et al. bekannt [J. Chabbat, M. Tellier, P. Porte und M. Steinbuch: Properties of a new fibrin glue stable in liquid state. Thromb. Res. 76: 525-533 (1994)].

Darüberhinaus ist auch schon in einer Publikation von D.V. Brezniak, H.I. Hassouna und J.W Fenton II [Blood Coagulation and Fibrinolysis, 6, 847-848 (1994)] der Einfluss von Salzen auf die Stabilität von verdünnten α-Thrombin-Lösungen beschrieben worden. Hier wurde gezeigt, dass in verdünnten Thrombin-Lösungen Natriumchlorid-Konzentrationen ab 0,3 Mol/l eine deutliche Stabilisierung bewirken. Danach soll Thrombin in Natriumchlorid-haltigen Lösungen für etwa 2 Wochen bei 37°C stabil sein und somit eine höhere Stabilität als in Calciumchlorid-haltigen Lösungen aufweisen, was möglicherweise durch eine bessere thermische Stabilität gegenüber einer Denaturierung erklärt werden kann.

In zahlreichen Patentanmeldungen sind auch bereits Verfahren zur Herstellung hochgereinigter Thrombin-Zubereitungen beschrieben worden. So ist aus der europäischen Patentanmeldung 0 439 156 ein Verfahren für die Herstellung eines gereinigten Thrombins mit einer spezifischen Aktivität größer als 1600 U/mg bekannt, das für die Hämostase eingesetzt werden kann. Dabei wird Thromboplastin zur Aktivierung von Prothrombin verwendet und eine Anionenaustausch- und eine Kationenaustauschchromatographie mit Trägermaterialien basierend auf Agarose eingesetzt. Ein "ultra-reines", klares, farbloses Rinder-Thrombin mit einer spezifischen Aktivität von ca. 8.000 bis 11.000 NIH U/mg wird in der US-Patentschrift 5 397 704 beschrieben. Zur Prothrombin-Aktivierung wird dort Thromboplastin aus Rinderlunge eingesetzt und zur Proteinreinigung werden Anionenaustausch- und Kationenaustauschchromatographie verwendet.

Keines der bisher bekannten Verfahren erlaubt jedoch die Herstellung einer gereinigten, Calcium-Ionen enthaltenden, virussicheren und im flüssigen Zustand bei 0°C und höheren Temperaturen stabilen Thrombin-Zubereitung, deren Thrombinaktivität nach 12 Monaten und darüber hinaus noch bei über 70-80 % des Ausgangswertes liegt. Es stellte sich deshalb die Aufgabe, ein Verfahren zur Herstellung einer derartigen Thrombin-Zubereitung zu entwickeln, wobei aus Gründen der Präparatesicherheit auf die Verwendung von Thromboplastin bei der Prothrombin-Aktivierung verzichtet werden sollte. Außerdem bestand die Aufgabe, hohe Konzentrationen von Polyolen als Stabilisatorzusatz zu vermeiden, weil dadurch eine unerwünschte Erhöhung der Viskosität der Zubereitung eintritt.

Es wurde nun gefunden, dass diese Aufgabe durch die Gegenstände, wie sie in den Ansprüchen beschrieben sind, gelöst wird. Ein Gegenstand der Erfindung ist ein Verfahren zur Herstellung einer Thrombin-Zubereitung, bei dem ein aus Plasma oder einer Plasmafraktion gewonnenes Prothrombin nach Aktivierung zu Thrombin ohne Zusatz von Thromboplastin sowie gegebenenfalls weiteren Aufarbeitungsschritten durch eine hydrophobe Interaktionschromatographie gereinigt wird und gegebenenfalls anschließend die Viren inaktiviert oder entfernt werden.

Eine weitere Verbesserung dieses Verfahrens ist möglich, wenn vor oder nach der hydrophoben Interaktionschromatographie zusätzlich noch eine Kationenaustauschchromatographie durchgeführt wird. Dabei können die Chromatographien als "positive" (Bindung des Thrombins) oder als "negative" Chromatographie (Bindung der Verunreinigungen) durchgeführt werden.

Da für die Erzielung einer hohen Thrombin-Stabilität im flüssigem Zustand eine ausreichende Reinheit der eingesetzten Thrombin-Lösung notwendig ist, wurde nach einem einfachen und verbesserten Verfahren gesucht, um hochreines Thrombin mit hoher Virussicherheit herzustellen. Ausgegangen werden kann dabei z.B. von dem in der europäischen Patentanmeldung 0 543 178 beschriebenen Verfahren zur Herstellung eines Thrombinkonzentrats. Aber auch andere Verfahren, bei denen teilgereinigtes Prothrombin in Anwesenheit von Calciumsalzen zu Thrombin aktiviert wird, können erfindungsgemäss als Ausgangsmaterial zur Herstellung der Thrombin-Zubereitung eingesetzt werden.

Setzt man nun zur Thrombin-Reinigung die hydrophobe Interaktionschromatographie (HIC) allein oder in Kombination mit einer Kationenaustauschchromatographie (CEC) ein, dann wird dadurch eine effektive und einfache Reinigung erreicht. Die Reihenfolge dieser beiden chromatographischen Verfahren ist dabei beliebig. Wird die Chromatographie zunächst mit einem hydrophoben Träger durchgeführt, kann das Thrombineluat anschließend direkt an den Kationenaustauscher gebunden und dort mittels eines Salzgradienten eluiert werden. Durch Kombination dieser beiden Trennprinzipien wird bei einer guten Ausbeute von etwa 70% über beide Reinigungsschritte eine Thrombin-Zubereitung hoher Reinheit erhalten.

Gleichzeitig wird dabei auch eine gute Abtrennung von Nebenprodukten wie aktivierten oder nicht-aktivierten Gerinnungsfaktoren und von im Gerinnungstest wenig oder nicht aktiven Thrombin-Formen (z.B. Prothrombin, β-Thrombin, γ-Thrombin oder anderen Thrombin- oder Prothrombinfragmenten) erreicht. Die Kombination der beiden genannten chromatographischen Verfahren ergibt eine höhere Reinheit als die alleinige Verwendung einer lonenaustauschchromatographie.

Das erfindungsgemäße Herstellungsverfahren wird so durchgeführt, dass zunächst Thrombin geringer oder mittlerer Reinheit hergestellt wird. Dies kann so erfolgen, dass die Adsorption von Prothrombin aus Plasma oder einer Plasmafraktion an einem Ionenaustauscher erfolgt. Das dadurch gewonnene Prothrombin kann dann einer Virusinaktivierung, z.B. mittels Pasteurisierung oder einer anderen bekannten Methode, und gegebenenfalls weiteren Aufarbeitungsschritten unterworfen und dann das Thrombin ohne Zusatz von aus tierischem Gewebe gewonnenen Thromboplastin nach an sich bekannten Verfahren aktiviert werden. Mit der sich dann anschließenden hydrophoben Interaktionschromatographie wird eine Abreicherung von begleitenden Plasmaproteinen, aktivierten Faktoren oder ihren Fragmenten sowie von Thrombin-Spaltprodukten erreicht. Dieser Reinigungseffekt wird durch die nachgeschaltete Kationenaustauschchromatographie weiter verstärkt.
Nach Elution des reinen Thrombins wird dann durch Zugabe geeigneter Puffersubstanzen der pH-Wert der Zubereitung auf den Bereich von 5 bis 8 eingestellt und Stabilisatoren zugesetzt. Der Zusatz von Puffersubstanz(en) und Stabilisatoren kann auch gemeinsam erfolgen.

Bei der als chromatographische Methode an sich bekannten hydrophoben Interaktionschromatographie wird als Adsorbens ein Gel mit gekoppelten, hydrophoben Resten eingesetzt. Besonders geeignete hydrophobe Reste sind hier Phenylreste oder andere Liganden mit einer ähnlichen Hydrophobizität.

Als Kationenaustauscher wird vorzugsweise ein Gel mit hoher Auflösung für die verschiedenen Thrombin-Varianten eingesetzt. Beispiele für geeignete Kationenaustauschergele sind Fractogel EMD SO₃ (Merck, Darmstadt), Macro Prep 50S (Biorad, München) oder andere Kationenaustauscher, die den heutigen Anforderungen bezüglich Reinigung und Sterilisierbarkeit entsprechen.

Die nach chromatographischer Reinigung gewonnene Thrombin-Lösung kann dann unmittelbar einer Virusinaktivierung oder einer Virusabreicherung wie z. B. einer Filtration über kleinporige Membranen zugeführt werden, wodurch eine effektive Abtrennung auch kleinster Viren unter Erzielung einer hohen Thrombinausbeute möglich ist. Eine Virusinaktivierung oder -abreicherung von Thrombin kann jedoch auch vor der chromatographischen Reinigung erfolgen, falls dadurch der Gesamtprozess erleichtert wird (z.B. durch Entfernung unerwünschter Komponenten oder Nebenprodukten in der nachfolgenden Chromatographie).

Zur Formulierung der Thrombin-Zubereitung als einer im flüssigen und gegebenenfalls auch im eingefrorenen Zustand stabilen und lagerfähigen Komponente für den Einsatz in einem Gewebekleber oder alleine als lokales Hämostyptikum sollte mit Hilfe eines Puffers ein pH-Wert von etwa 5,0 bis 8,0 eingestellt werden. Zur Erzielung des gewünschten Effektes bei der Anwendung bzw. zur Stabilisierung werden dann der Zubereitung ein lösliches Calciumsalz, Natriumchlorid, ein Zucker oder ein Zuckeralkohol und/oder eine Aminosäure oder auch das Salz einer Mono- oder Polycarbonsäure und/oder das Salz einer Mono- oder Polyhydroxycarbonsäure zugesetzt. Damit ergeben sich gute Stabilitäten im flüssigen und/oder gefrorenen Zustand für 12 Monate Lagerzeit und darüber hinaus.

Es hat sich auch gezeigt, dass durch Zusatz von die Thrombin-Aktivität in vitro nicht-kovalent inhibierenden Substanzen die Stabilität vor allem bei Raumtemperatur noch weiter signifikant erhöht werden kann, indem die Autolyse des Thrombins zurückgedrängt wird. Geeignete Substanzen hierfür sind Verbindungen wie Benzamidin oder p-Aminobenzamidin oder andere niedrig- bis mittelaffine Protease-Inhibitoren. Durch Zusatz dieser niedrig- oder mittelaffinen Inhibitoren wird die Aktivität von Thrombin gegenüber Substanzen wie Fibrinogen nicht wesentlich beeinträchtigt und dadurch auch nicht z.B. der spätere Einsatz als Komponete eines Gewebeklebers.

Mit den erfindungsgemäßen Verfahren werden Thrombin-Zubereitungen herstellbar, die in flüssigem und/oder gefrorenen Zustand über Monate oder Jahre gelagert werden können und deren Aktivität in dieser Zeit nicht unter 70-80% abfällt.

Mit den erfindungsgemäßen Verfahren ist es möglich, auch in Anwesenheit von Calciumsalzen, die die thermische Stabilität von Thrombin erniedrigen [B.H. Landis, K.A. Koehler und J.W. Fenton II: Human Thrombins. J Biol chem 256: 4604-4610 (1981)], Thrombin-Zubereitungen herzustellen, die bis zu 24 Monaten und darüber hinaus bei 4°C eine hohe Stabilität ergeben, wie im Gerinnungstest nachweisbar ist. Auch im gefrorenen Zustand sind viele der in Tabelle 4 gezeigten Thrombin-Zubereitungen stabil und selbst bei Raumtemperatur ist die Stabilität in den meisten Fällen für einen Zeitraum von 3 bis 6 Monaten gegeben. Bei Raumtemperatur lässt sich die Stabilität im Besonderen durch den Zusatz niedrig- oder mittelaffiner Thrombin-Inhibitoren wie z.B. Benzamidin, p-Aminobenzamidin oder anderer Protease- bzw. Thrombin-Inhibitoren erhöhen, ohne dass dabei die Aktivität gegenüber Fibrinogen im Gerinnungstest signifikant abfällt.

Die nach den beschriebenen Verfahren hergestellten Thrombin-Zubereitungen können u.a. als Komponenten eines im flüssigen oder eingefrorenen Zustand lagerfähigen Fibrinklebers, bestehend aus zwei Komponenten, z.B. einer Thrombin- und einer Fibrinogen-haltigen Komponente, oder bestehend aus drei Komponenten, z.B. einer Thrombin-, Fibrinogen- und einer Faktor XIII-haltigen Komponente, eingesetzt werden, wie u.a. in der deutschen Patentanmeldung 198 53 033.1 beschrieben. Dabei kann die so hergestellte Thrombin-Zubereitung entweder in situ mit den anderen Komponenten vermischt werden oder sie kann im Falle eines 3-Komponenten Fibrinklebers auch vorab mit einer der Komponenten vermischt werden, bevor beide der dritten Komponente zugegeben werden. Möglich ist aber auch die Herstellung von lyophilisierten Thrombin-Zubereitungen unter Verwendung des erfindungsgemäßen Verfahrens für therapeutische Zwecke, wobei nach Rekonstitution im flüssigen Zustand noch eine entsprechend hohe Stabilität beobachtet wird.

Schließlich können die erfindungsgemäß hergestellten Thrombin-Konzentrate auch allein oder in Kombination mit Trägermaterialien als Wirkstoff zur lokalen Blutstillung eingesetzt werden.

Das erfindungsgemäße Verfahren wird durch die nachfolgenden Beispiele näher erläutert:

### Beispiel 1: Thrombinreinigung

Ausgehend von einem Thrombin-Konzentrat geringer oder mittlerer Reinheit, hergestellt nach bekannten Verfahren, wurden zwei Chromatographieschritte durchgeführt.

Zunächst wurde die Thrombinlösung mit 0,6 Mol/l Natriumsulfat versetzt und an einem hydrophoben Chromatographiegel (hier: Phenyl-Sepharose HP, Hersteller: Amersham Pharmacia, Freiburg, Deutschland) adsorbiert, das zuvor mit Puffer A (10 mMol/l Na-Phosphat pH 6,5) enthaltend 0,6 Mol/l Natriumsulfat äquilibriert worden war. Nach Waschen mit Puffer A enthaltend 0,6 Mol/l Natriumsulfat wurde durch einen Gradienten mit abfallendem Natriumsulfat-Gehalt in Puffer A das gebundene Thrombin eluiert. Verunreinigungen und Thrombinfragmente wurden zu einem großen Teil im Durchlauf oder in den Waschfraktionen abgetrennt.

Die Thrombin-Fraktion wurde ohne weitere Behandlung direkt auf eine mit Puffer A äquilibrierte Kationentauschersäule (hier: Fractogel^{®} EMD SO_{3,} Hersteller: Merck, Darmstadt, Deutschland) gegeben, mit Äquilibrierungspuffer A gewaschen und durch einen Gradienten von 0 bis 1,0 Mol/l Natriumchlorid in Puffer A eluiert. Im Verlaufe der Trennung werden letzte Nebenprodukte und Thrombin-Fragmente abgetrennt, so dass das erhaltene α-Thrombin-Eluat eine hohe spezifische Reinheit von ca. 3500 IU/mg aufwies [Proteinbestimmung mittels Bestimmung der Absorption bei 280 nm und Umrechnung mit dem Faktor von 1,74 für eine 0,1%ige Lösung nach J.W. Fenton, II, M.J. Fasco, A.B. Stackrow, D.L. Aronson, A.M. Young, and J.S. Finlayson. Human Thrombins. J Biol Chem 252: 3587-3598 (1977)]. Tabelle 1 zeigt die Ergebnisse dieser Thrombin-Reinigung und die erhaltene spezifische Aktivität.

Auf dieser Stufe kann das Thrombin in gekühltem oder tiefgekühltem Zustand bis zur weiteren Verarbeitung gelagert werden.

**Tabelle 1:**

| Probe | Absorption 280 nm | Protein* (mg/ml) | Aktivität (IU/ml) | Spezifische Aktivität (IU/mg) |
|---|---|---|---|---|
| Thrombin, Ausgangsmat. | 13,78 | 7,92 | 6418 | 810 |
| HIC-Eluat | 1,085 | 0,624 | 1372 | 2199 |
| CEC-Eluat | 6,65 | 3,822 | 13370 | 3498 |

| | | | | |
|---|---|---|---|---|
| * A_{280,0,1%} =1,74 | | | | |

### Beispiel 2: Thrombinreinigung

Ausgehend von einem Thrombin-Konzentrat mittlerer oder geringer Reinheit wurden zwei Chromatographieschritte durchgeführt. Zunächst wurde die Thrombin-Lösung mit 0,6 Mol/l Natriumsulfat versetzt und an einem hydrophoben Chromatographiegel (hier: Phenyl-Sepharose HP, Hersteller; Amersham Pharmacia, Freiburg, Deutschland) adsorbiert, das zuvor mit Puffer B (10 mMol/l Na-Phosphat, 0,1% PEG pH 6,5 [hier PEG 6000, aber auch andere Molekulargewichtsbereiche sind einsetzbar]) enthaltend 0,6 Mol/l Natriumsulfat äquilibriert worden war. Nach Waschen mit Puffer B enthaltend 0,6 Mol/l Natriumsulfat wurde durch einen Gradienten mit abfallendem Natriumsulfat-Gehalt in Puffer B das gebundene Thrombin eluiert. Verunreinigungen und Thrombinfragmente wurden zu einem großen Teil im Durchlauf oder in den Waschfraktionen abgetrennt.

Die Thrombin-Fraktion wurde ohne weitere Behandlung direkt auf eine mit Puffer C (10 mMol/l Na-Phosphat, 166 mMol/l L-Arginin pH 6,5) äquilibrierte Kationentauschersäule (hier: Fractogel^{®} EMD SO₃, Hersteller: Merck, Darmstadt, Deutschland) gegeben, mit Äquilibrierungspuffer C gewaschen und durch einen Gradienten von 0 bis 1,0 Mol/l Natriumchlorid in Puffer C eluiert. Im Verlaufe der Trennung werden noch vorhandene Nebenprodukte und Thrombin-Fragmente abgetrennt, so dass das erhaltene α-Thrombin-Eluat eine hohe spezifische Reinheit von ca. 3300 IU/mg aufwies (vgl. Tabelle 2).

Auf dieser Stufe kann das erhaltene Thrombin in gekühltem oder tiefgekühltem Zustand bis zur weiteren Verarbeitung gelagert werden.

**Tabelle 2:**

| Probe | Absorption 280 nm | Protein* (mg/ml) | Aktivität (IU/ml) | Spezifische Aktivität (IU/mg) |
|---|---|---|---|---|
| Thrombin, Ausgangsmat. | 12,49 | 7,178 | 5895 | 821 |
| HIC-Eluat | 2,042 | 1,174 | 2696 | 2296 |
| CEC-Eluat | 8,03 | 4,615 | 15150 | 3283 |

| | | | | |
|---|---|---|---|---|
| *A_{280,0,1%} = 1,74 | | | | |

### Beispiel 3: Thrombinreinigung

Entsprechend Beispiel 1 wurde eine Thrombinreinigung durchgeführt, jedoch mit der Abweichung, dass der bei der Chromatographie engesetzte Puffer anstelle von Natriumphosphat 20 mMol/l L-Histidin enthält. Diese Modifikation ergibt vergleichbare Reinigungsergebnisse wie in Beispiel 1, kann aber die weitere Aufarbeitung zum Endprodukt vereinfachen, wenn hier z.B. Histidin als Puffersubstanz enthalten sein soll.

### Beispiel 4: Thrombinreinigung und Filtration

Ausgehend von einem wie in den Beispielen 1 bis 3 gereinigten Thrombin-Eluat wurde nach hydrophober Interaktionschromatographie und Kationenaustauschchromatographie eine Filtration an einer Membran mit kleiner Porengröße durchgeführt (z.B. Pianova^{™} 15 nm). Mit Hilfe dieser Membran lassen sich selbst kleine Viren wie Parvoviren effektiv abtrennen. Es wurde gefunden, dass bei Verwendung des gereinigten Thrombins als Ausgangsmaterial bei einer guten Filtrationsrate sehr gute Ausbeuten bezüglich Thrombinaktivität und Protein erhalten wurden (siehe Tabelle 3). Dadurch ist dieses Verfahren geeignet zur Herstellung eines Thrombin-Konzentrates mit hohen Abreicherungsfaktoren für Viren.

**Tabelle 3: Filtration von 123 ml gereinigtem Thrombin über ein Pianova^{™} 15 nm Modul (0,001 m²)**

| **Probe** | Thrombin-Aktivität, gesamt | Protein, gesamt* |
|---|---|---|
| vor Filtration | 800.240 IU | 245,3 mg |
| nach Filtration | 797.960 IU | 239,0 mg |
| Ausbeute | 99,7% | 97,4% |

| | | |
|---|---|---|
| * A_{280,0,1%} = 1,74 | | |

### Beispiel 5: Thrombin-Formulierungen

Ausgehend von chromatographisch gereinigtem Thrombin wurden verschiedene Formulierungen hergestellt und bei Temperaturen von -20°C, 4°C, 20-25°C und teilweise auch bei 37°C gelagert. Die Herstellung dieser Thrombinlösungen erfolgte durch Diafiltration der gereinigten Thrombinkonzentrate gegen den Formulierungspuffer oder durch Diafiltration gegen einen Grundpuffer und Zusatz der restlichen Additive, pH-Einstellung und Einstellung der Thrombin-Konzentration. Thrombin-Konzentrationen von ca. 1 bis zu ca. 15.000 IU/ml können auf diese Weise hergestellt werden.

Durch Bestimmung der Thrombinaktivität in einem Gerinnungstest mit Fibrinogen als Substrat wurden die Formulierungen auf ihre Stabilität getestet. Tabelle 4 zeigt eine Auswahl der erfindungsgemäßen Formulierungen und ihre Stabilisatorzusammensetzung und Tabelle 5 zeigt die entsprechenden Stabilitätsdaten bei bis zu drei Temperaturen.

### Tabelle 4: Zusammensetzung von Thrombinformulierungen

1. 360 mMol/l NaCl, 40 mMol/l CaCl₂, 5 mMol/l L-Histidin pH 6,0
2. 360 mMol/l NaCl, 40 mMol/l CaCl₂, 2% (w/v) Mannitol, 5 mMol/l L-Histidin pH 6,0
3. 150 mMol/l NaCl, 40 mMol/l CaCl₂, 2% (w/v) Mannitol, 5 mMol/l L-Histidin pH 6,0
4. 90 mMol/l NaCl, 40 mMol/l CaCl₂, 100 mMol/l Na-Succinat, 5 mMol/l L-Histidin pH 6,0
5. 90 mMol/l NaCl, 40 mMol/l CaCl₂, 100 mMol/l Na-Succinat, 2% (w/v) Mannitol, 5 mMol/l L-Histidin pH 6,0
6. 150 mMol/l NaCl, 40 mMol/l CaCl₂, 100 mMol/l Na-Succinat, 5 mMol/l L-Histidin pH 6,0
7. 90 mMol/l NaCl, 40 mMol/l CaCl₂, 50 mMol/l Na-Laktat, 2% (w/v) Mannitol, 5 mMol/l L-Histidin pH 6,0
8. 90 mMol/l NaCl, 40 mMol/l CaCl₂, 2% (w/v) Mannitol, 10 mMol/l p-Aminobenzamidin, 5 mMol/l L-Histidin pH 6,0
9. 90 mMol/l NaCl, 40 mMol/l CaCl₂, 2% (w/v) Mannitol, 10 mMol/l Benzamidin, 5 mMol/l L-Histidin pH 6,0
10. 90 mMol/l NaCl, 40 mMol/l CaCl₂, 4% (w/v) HSA, 5 mMol/l L-Histidin pH 6,0
11. 90 mMol/l NaCl, 40 mMol/l CaCl₂, 1 % (w/v) Mannitol, 142 mMol/l L-Arginin, 5 mMol/l L-Histidin pH 6,0
12. 90 mMol/l NaCl, 40 mMol/l CaCl₂, 100 mMol/l Na-Succinat, 0,1% Polyvinylpyrrolidon (K15), 5 mMol/l L-Histidin pH 6,0.

**Tabelle 5: Stabilität von Thrombin in verschiedenen Formulierungen bei 4°C, -20°C und 20-25°C**

| Thrombinaktivität (% vom Nullwert), Lagertemperatur: 4°C | | | | | | | | | | | | |
|---|---|---|---|---|---|---|---|---|---|---|---|---|
| Lagerzeit (Monate) | Ansatz | | | | | | | | | | | |
| | 1 | 2 | 3 | 4 | 5 | 6 | 7 | 8 | 9 | 10 | 11 | 12 |
| 0 | 100 | 100 | 100 | 100 | 100 | 100 | 100 | 100 | 100 | 100 | 100 | 100 |
| 1 | 101.5 | 95.8 | 98.9 | 94,0 | 100.4 | 100,8 | 110.6 | 102 | 98,0 | 106.8 | 108.3 | 98,0 |
| 3 | 98.9 | 109.8 | 103.4 | 103,1 | 108.8 | 104,5 | 101.6 | 95,6 | 91,5 | 98.5 | 102.3 | 103,9 |
| 6 | 100.5 | 117.5 | 88.4 | 97,3 | 99,0 | 98,2 | 108.1 | 97,6 | 95.2 | 103.0 | 99.4 | 101,9 |
| 9 | 97.5 | 112.9 | 95.6 | 94,2 | 91,9 | 91,6 | 122.7 | 102,2 | 99,6 | 102.2 | 91.5 | 108,0 |
| 12 | 100.2 | 116,5 | 93.6 | 92,4 | 105,8 | 106,4 | 117,2 | 100,2 | 97.8 | 101.2 | 93.5 | 103,8 |
| 18 | 89.7 | 101,7 | - | 94,7 | | | 112,7 | - | - | 89.2 | 95.8 | 104,1 |
| 24 | 100.5 | | 86.8 | 95,5 | | | | 100 | 94,3 | 89.2 | 96.2 | |

| Thrombinaktivität (% vom Nullwert), Lagertemperatur: -20°C | | | | | | | | | | | | |
|---|---|---|---|---|---|---|---|---|---|---|---|---|
| Lagerzeit (Monate) | Ansatz | | | | | | | | | | | |
| | 1 | 2 | 3 | 4 | 5 | 6 | 7 | 8 | 9 | 10 | 11 | 12 |
| 0 | 100 | | 100 | 100 | 100 | 100 | | | 100 | 100 | 100 | |
| 1 | 103.6 | | 100.4 | 88.6 | 94.3 | 94,9 | | | 47,1 | 111.0 | 106.5 | |
| 3 | 97.5 | | 92.6 | 104.2 | 93.0 | 93,9 | | | 91,5 | 103.0 | 101.3 | |
| 6 | 99.5 | | 92.7 | 101.3 | 99,7 | 98,2 | | | 68,6 | 107.3 | 96.4 | |
| 9 | 100.3 | | 81.3 | 92.9 | 89,2 | 87,6 | | | 92,1 | 108.0 | 95.3 | |
| 12 | 94.3 | | 100.5 | 95.3 | 104,4 | 98,5 | | | 79,2 | 104.0 | 95.1 | |
| 18 | 94.3 | | - | 92.7 | | | | | - | 99.3 | 99.8 | |
| 24 | 99.0 | | 90.7 | 100.7 | | | | | 97,2 | 104.2 | 100.2 | |

| Thrombinaktivität (% vom Nullwert), Lagertemperatur: 20-25°C | | | | | | | | | | | | |
|---|---|---|---|---|---|---|---|---|---|---|---|---|
| Lagerzeit (Monate) | Ansatz | | | | | | | | | | | |
| | 1 | 2 | 3 | 4 | 5 | 6 | 7 | 8 | 9 | 10 | 11 | 12 |
| 0 | 100 | 100 | 100 | 100 | 100 | 100 | 100 | 100 | 100 | 100 | 100 | 100 |
| 1 | 105.6 | 92,4 | 90.9 | 91.8 | 92,6 | 94,3 | 95.5 | 101,7 | 99,8 | 107.3 | 99.4 | 81,1 |
| 3 | 92.3 | 88.2 | 86.9 | 88.4 | 80.0 | 77,9 | 75.8 | 95,4 | 96,3 | 84.2 | 98.9 | 77,0 |
| 6 | 85.6 | 66.3 | 66.8 | 80.4 | 71,4 | 68,6 | 75.3 | 92,8 | 89,0 | 69.4 | 84.8 | 60,6 |
| 9 | 72.1 | 58.3 | 58.8 | 75.9 | 51,8 | 50,7 | 59.1 | 96,1 | 91,9 | 48.8 | 74.4 | 53,3 |
| 12 | 64.2 | 50,1 | 51.5 | 65.3 | 43,7 | 43,9 | 42,8 | 100,9 | 90,6 | 42.9 | 64.7 | 46,6 |
| 18 | | | | | | | | - | - | | | |
| 24 | | | | | | | | 90,1 | 82,4 | | | |

## Patentansprüche

1. Verfahren zur Herstellung einer Thrombin-Zubereitung, wobei ein aus Plasma oder einer Plasmafraktion gewonnenes Prothrombin nach Aktivierung zu Thrombin ohne Zusatz von Thromboplastin durch eine hydrophobe Interaktionschromatographie gereinigt wird.

2. Verfahren nach Anspruch 1, **dadurch gekennzeichnet, dass** vor oder nach der hydrophoben Interaktionschromatographie zusätzlich noch eine Kationenaustauschchromatographie durchgeführt wird.

3. Verfahren nach Anspruch 1 oder 2, **dadurch gekennzeichnet, dass** nach der hydrophoben Interaktionschromatographie und der Kationenaustauschchromatographie eine Filtration an einer Membran mit kleiner Porengröße durchgeführt wird.

4. Verfahren zur Herstellung einer Thrombin-Zubereitung nach einem der Ansprüche 1 bis 3, **dadurch gekennzeichnet, dass** das für die Aktivierung zu Thrombin eingesetzte Prothrombin im Rahmen seiner Herstellung einer Virusinaktivierung oder Abreicherung unterworfen wird.

5. Verfahren zur Herstellung einer Thrombin-Zubereitung nach einem der Ansprüche 1 bis 4, **dadurch gekennzeichnet, dass** das Thrombin vor oder nach der chromatographischen Reinigung noch einer zusätzlichen Inaktivierung oder Abreicherung von Viren unterworfen wird.

6. Verfahren nach einem der Ansprüche 1 bis 5, **dadurch gekennzeichnet, dass** die Thrombin-Zubereitung auf einen pH-Wert von 5,0 bis 8,0 eingestellt wird.

7. Verfahren nach den Ansprüchen 1 bis 6, **dadurch gekennzeichnet, dass** der Thrombin-Zubereitung neben einem löslichen Calciumsalz und Natriumchlorid als Stabilisatoren
- eine Puffersubstanz,
- ein Zucker oder Zuckeralkohol und/oder eine Aminosäure und/oder
- ein Salz einer Mono- oder Polycarbonsäure oder
- ein Salz einer Mono- oder Polyhydroxycarbonsäure
zugesetzt werden.

8. Verfahren nach den Ansprüchen 1 bis 7, **dadurch gekennzeichnet, dass** als Stabilisator ein niedrig bis mittelaffiner Proteaseinhibitor zugesetzt wird.

9. Verfahren nach Anspruch 8, **dadurch gekennzeichnet, dass** als Proteaseinhibitor Benzamidin oder p-Aminobenzamidin zugesetzt wird.

10. Im flüssigen Zustand stabile Thrombin-Zubereitung, **dadurch gekennzeichnet, dass** sie als die Thrombinaktivität inhibierende Substanz Benzamindin oder p-Aminobenzamidin enthält und dass sie neben einem löslichen Calciumsalz und Natriumchlorid als Stabilisatoren
- eine Puffersubstanz,
- ein Zucker oder Zuckeralkohol und/oder eine Aminosäure und/oder
- ein Salz einer Mono- oder Polycarbonsäure oder
- ein Salz einer Mono- oder Polyhydroxycarbonsäure
enthält.

11. Eine Thrombin-Zubereitung nach Anspruch 10 zur Verwendung als Hämostypticum, als Bestandteil eines Hämostypticums oder als Bestandteil eines Gewebeklebers.

## Claims

1. Process for producing a thrombin preparation, wherein a prothrombin obtained from plasma or a plasma fraction is, after activation to thrombin without the addition of thromboplastin, purified by a hydrophobic interaction chromatography.

2. Process according to Claim 1, **characterized in that** a cation-exchange chromatography is additionally carried out before or after the hydrophobic interaction chromatography.

3. Process according to Claim 1 or **2, characterized in that** a filtration is carried out on a membrane of small pore size after the hydrophobic interaction chromatography and the cation-exchange chromatography.

4. Process for producing a thrombin preparation according to any of Claims 1 to 3, **characterized in that** the prothrombin used for the activation to thrombin is subjected to a virus inactivation or depletion during the production thereof.

5. Process for producing a thrombin preparation according to any of Claims 1 to 4, **characterized in that** the thrombin is additionally subjected to an additional virus inactivation or depletion before or after the chromatographic purification.

6. Process according to any of Claims 1 to 5, **characterized in that** the thrombin preparation is adjusted to a pH of from 5.0 to 8.0.

7. Process according to Claims 1 to 6, **characterized in that** besides a soluble calcium salt and sodium chloride
- a buffer substance,
- a sugar or sugar alcohol and/or an amino acid and/or
- a salt of a mono- or polycarboxylic acid or
- a salt of a mono- or polyhydroxycarboxylic acid
are added to the thrombin preparation as stabilizers.

8. Process according to Claims 1 to 7, **characterized in that** a low to moderate affinity protease inhibitor is added as stabilizer.

9. Process according to Claim 8, **characterized in that** benzamidine or p-aminobenzamidine is added as protease inhibitor.

10. Thrombin preparation stable in the liquid state, **characterized in that** it contains benzamidine or p-aminobenzamidine as substance inhibiting the thrombin activity and **in that** it contains besides a soluble calcium salt and sodium chloride
- a buffer substance,
- a sugar or sugar alcohol and/or an amino acid and/or
- a salt of a mono- or polycarboxylic acid or
- a salt of a mono- or polyhydroxycarboxylic acid
as stabilizers.

11. Thrombin preparation according to Claim 10 for use as haemostatic, as constituent of a haemostatic or as constituent of a tissue glue.

## Revendications

1. Procédé pour la production d'une préparation de thrombine, une prothrombine obtenue à partir de plasma ou d'une fraction plasmatique étant purifiée après activation en thrombine sans addition de thromboplastine par une chromatographie d'interaction hydrophobe.

2. Procédé selon la revendication 1, **caractérisé en ce qu'**on réalise, avant ou après la chromatographie d'interaction hydrophobe, en plus encore une chromatographie d'échange cationique.

3. Procédé selon la revendication 1 ou 2, **caractérisé en ce qu'**on réalise, après la chromatographie d'interaction hydrophobe et la chromatographie d'échange cationique, une filtration sur une membrane présentant une petite taille de pores.

4. Procédé pour la production d'une préparation de thrombine selon l'une quelconque des revendications 1 à 3, **caractérisé en ce que** la prothrombine utilisée pour l'activation en thrombine est soumise à une inactivation ou un appauvrissement en virus dans le cadre de sa production.

5. Procédé pour la production d'une préparation de thrombine selon l'une quelconque des revendications 1 à 4, **caractérisé en ce que** la thrombine, avant ou après la purification par chromatographie, est encore soumise à une inactivation ou un appauvrissement supplémentaire en virus.

6. Procédé selon l'une quelconque des revendications 1 à 5, **caractérisé en ce que** la préparation de thrombine est réglée à un pH de 5,0 à 8,0.

7. Procédé selon les revendications 1 à 6, **caractérisé en ce que** la préparation de thrombine est additionnée, outre d'un sel de calcium soluble et de chlorure de sodium, comme stabilisateurs,
- d'une substance tampon,
- d'un sucre ou d'un alcool de sucre et/ou d'un acide aminé et/ou
- d'un sel d'un acide monocarboxylique ou polycarboxylique ou
- d'un sel d'un acide monohydroxycarboxylique ou polyhydroxycarboxylique.

8. Procédé selon les revendications 1 à 7, **caractérisé en ce qu'**on ajoute, comme stabilisateur, un inhibiteur de protéase présentant une affinité faible à moyenne.

9. Procédé selon la revendication 8, **caractérisé en ce qu'**on utilise, comme inhibiteurs de protéase, la benzamidine ou la p-aminobenzamidine.

10. Préparation de thrombine stable à l'état liquide, **caractérisée en ce qu'**elle contient, comme substance inhibant l'activité de la thrombine, de la benzamidine ou de la p-aminobenzamidine et **en ce qu'**elle contient, outre un sel soluble de calcium et du chlorure de sodium, comme stabilisateurs,
- une substance tampon,
- un sucre ou un alcool de sucre et/ou un acide aminé et/ou
- un sel d'un acide monocarboxylique ou polycarboxylique ou
- un sel d'un acide monohydroxycarboxylique ou polyhydroxycarboxylique.

11. Préparation de thrombine selon la revendication 10 pour une utilisation comme hémostyptique ou comme constituant d'un adhésif tissulaire.
